# EUROPEAN PATENT APPLICATION

(11) **EP 0 797 998 A1**
(43) Date of publication of application: **01.10.1997**
(21) Application number: 96938486.6
(22) Date of filing: 15.11.1996
(51) Int. Cl.: A61K 38/21

(54) **ENDOTHELIAL CELL PROTECTIVE**

(30) Priority: 17.11.1995 JP 300190/95
(71) Applicant: TORAY INDUSTRIES, INC., Tokyo 103 (JP)
(72) Inventor: SANO, Emiko, Yokohama-shi, Kanagawa 241 (JP)
(74) Representative: Kador & Partner
(86) International application number: JP9603366
(87) International publication number: WO9718831

(57) **Abstract**

An endothelial cell protective comprising an interferon as the active ingredient. It is used for the prevention and treatment of circulatory diseases, diabetic angiopathy, or vascular lesion in collagen diseases, or for healing wounds.

## Description

### TECHNICAL FIELD

The present invention relates to an agent for protecting endothelial cells comprising as an effective ingredient interferon (hereinafter, abbreviated as IFN).

### BACKGROUND ART

Vascular endothelial cells are cells which have an epithelium-like form, and cover, as a single layer, the intimal surface of various blood vessels. The vascular endothelial cells are deeply involved in the homeostasis of a body through their functions, such as maintenance of vascular fluidity, control of substance permeation, regulation of vascular wall tension, and the like. Since an isolation and culture methods for endothelial cells were established, various studies using cultured endothelial cells have been done along with development of molecular or cellular biology, revealing many things regarding the etiologies of, for example, arteriosclerosis, inflammation, thrombosis, blood pressure anomaly, cancer growth, and the like.

In recent years it has become known that the growth rate, form and functions of endothelial cells are changed by physical stimulation caused by blood flow. Dewey et al. (J. Biochem. Eng., 103, 177-184, 1981) have confirmed that endothelial cells form a line with long cell shape alongside a blood flow using cultured endothelial cells. Ando et al. (Microvasc. Res., 33, 62-70, 1987) have found that the growth rate and migration functions of endothelial cells are enhanced due to constant or pulsating flow load. It has been also reported that the production of physiologically active substances from endothelial cells is also changed due to the sheer stress by blood flow. Endothelial cells produce prostacyclin, tissue plasminogen activator, vasorelaxing factor (EDRF), prostaglandin F2α, endothelin, and the like, which have important effects on the homeostasis of blood vessels. The production of these substances has been reported to be controlled by force of blood flow (Tissue Culture, Vol. 20, No. 8, 2-7, 1994). It has been considered that thrombus frequently occurs in vessel branching portions and vessel constricted portions because disturbance of blood flow at such sites changes functions of endothelial cells.

Thus it has become well known that endothelial cells have important effects on maintenance of the normality of blood vessels, and that a function loss caused by vascular rhexis or damage can be a beginning of any vascular disease. However, there is no medicine that has a good protective effect on the rhexis of endothelial cells, and it is desired that a good preventive and curative medicine for vascular lesions be developed.

Disorder in blood circulation, particularly, myocardial infarction and brain infarction, are a major cause of deaths in recent years. In ischemic diseases developed after myocardial infarction or brain infarction, it is known that active oxygen produced at the time of reperfusion causes tissue injury. In particular, endothelial cell injury is considered a center part of development of ischemic damage (Olafsson B et al., irculation, 76, 1135-1145, 1987) (Forman MB et al., 76, 469-479, 1987). It is known that endothelial cells produce prostacyclin (PGl2) for maintaining the homeostasis against the radicals or lipoperoxides produced by leukocytes or macrophages.

Percutaneous coronary artery reconstruction (PTCA) is a high success rate therapy for stenocardia or acute mycardial infarction. However, application of this therapy is made difficult by vascular restenosis that frequently occurs after coronary artery reperfusion. A greatest cause of arteriosclerotic intima hyperplasia or restenosis is considered to be vascular endothelial injury. It has been reported that rhexis of endothelial cells causes migration of smooth muscle cells to intima from media layer of vascular wall and proliferation in an intima layer, thereby forming an intima hyperplasia. To reduce intima hyperplasia and restenosis, general pharmacotherapies have been tried, for example, an antiplatelet agent, an anticoagulant agent, a corticosteroid, a calcium channel blocking agent, and the like. However, no agent has been reported to be effective. This is because the intima hyperplasia and restenosis progress in combined complicate mechanisms. The prevention and cure thereof are expected to be achieved by development of a medicine that controls the injury of endothelial cells and sustains the endothelium function.

Diabetes is a representative disease based on insulin hyposecretion or insulin action disorder. Since the QOL and life prognosis of diabetic patients are dependent on vascular complications, diabetes is also considered a vascular disease. Diabetic vascular complications are classified into great vessel injury and small vessel injury. The great vessel injury is not a complication typical of diabetes, but it is considered to be caused due to development of an arteriosclerotic lesion in an early period of diabetes. The small vessel injury is typical of diabetes, and it is a complication that mainly include retinopathy and nephropathy (Kidney and Dialysis, Vol. 36, No. 2, 231-235, 1994).

With diabetes, the frequency of arteriosclerotic lesions, such as cardiovascular injury and peripheral vessel injury, is high. Lipids metabolism anomaly is being mainly studied for an onset mechanism of such arteriosclerotic lesions. A so-called insulin resistance syndrome that includes hyperinsulinemia, high blood pressure, hypertriglyceridemia and the like, is drawing attention, particularly, as a risk factor of ischemic heart diseases. In diabetic conditions, there is a high possibility that aortic smooth muscle cells have been transformed from a relaxation type to a contraction type, so that vasoconstriction or the like is likely to occur. The progress of these pathological forms is caused by rhexis of vascular endothelial cells.

Diabetic retinopathy, which is a micro vessel injury, is the number one primary disease of blindness of adults, and a serious complication that degrades the QOL of diabetic patients. As primary changes in retinopathy, capillary vessel-constituting cells that include endothelial cells undergo rhexis and deciduation, and hyperplasia of a capillary basal membrane is observed. As retinopathy progresses, neovascularity develops. The onset of diabetic retinopathy is greatly associated with deciduation of endothelial cells and loss of functions. The diabetic nephropathy, on the other hand, is the number two primary disease of chronic kidney failure patients introduced to dialysis, taking up 30% of the cases newly introduced thereto in the year of 1993.

Since nephropathy patients have very bad life prognosis after introduction of dialysis, development of a medicine that inhibits the onset and progress thereof is strongly desired. The nephropathy lesion occurs in glomerule, wherein injury of endothelial cells of capillary vessels filling the interior first occurs, and hyperplasia of the basal membrane of glomerule and multiplication of mesangial cells are histological features.

In this manner, in diabetes, the characteristic metabolism anomaly causes injury of vascular endothelial cells and, therefore, loss of the functions. Since this injury is greatly related to occurrence of a diabetic vascular lesion, a useful medicine that protects endothelial cells injury and sustain their functions is desired.

High blood pressure is clinically known as a risk factor that causes lesions relevant to vessels, such as cerebral hemorrhage, ischemic heart disease, aortic aneurysm, nephrosclerosis, and the like. It was conventionally considered that blood vessels were mere pipes for circulating blood, and the hypertensive vascular lesion formation was a result of blood platelet deposition or cholesterol deposition on injured endothelium, smooth muscle cell necrosis, and the like. However, it has recently been found that the endothelial cells and smooth muscle cells that form vessels produce various vasoactive substances and cytokine, and play important parts in vascular wall reconstruction such as arterial sclerosis or self-circulation regulation (Nakamura, Y. et al., CIRCULATION, 88, l-184, 1993).

Such changes in the vascular wall form and function are reported to be accelerating factors of high blood pressure (Folkow, B., Hypertension: Pathophysiology, Diagnosis and Management (Laragh, J. H. & Bronnar, B. M. eds) p565-581 Ravan Press Ltd., NY 1990). It can be considered that influences of blood pressure on vascular form and function and influences on high blood pressure caused by changes in vessels interact with each other. However, since it is endothelial cells that are initially affected by a blood pressure change, development of a good medicine having an endothelial cell protective effect is expected to have good preventive and curative effects on vascular injury caused by high blood pressure.

Collagen disease, considered to be an intractable disease, is an inflammatory disease accompanied by vasculitis syndrome. The angiopathic mechanism of the collagen disease is hardly elucidated. However, as histopathological appearances of vascular lesions normally termed angiitis, there are found vascular media fibrinoid degeneration and necrosis, and eosinophil leukocytic infiltrate, lymphocytic and monocytic infiltrate, and polymorphonuclear leukocytic infiltrate to vascular walls, and vascular intima hyperplasia, clot formation and aneurysm formation are subsequently observed. All of these vascular lesions are commonly triggered by endothelial cell injury. If endothelial cells are protected while they retain normal functions, preventive and curative effects on the angiitis in collagen disease can also be expected.

There presently is no effective curative agent for the above-described various vascular lesions, while there are many patients with those lesions. Therefore, development of an excellent medicine therefor is desired.

In the process of wound healing, there are complicate biological reactions that include heterocellular interactions, a growth factor, cytokine, extracellular matrix and the like. For the purpose of wound healing, epidermal growth factor (EGF), basic fibroblast growth factor (bFGF), antihemophilic factor, transforming growth factor (TGF) β2, 3, platelet-deriving growth factor (PDGF) and the like have been researched and developed. While the healing process is complicated, an enhanced drug effect can be expected if substances having different acting mechanisms are combined.

IFN is a protein produced by animal cells that have received a stimulus such as virus or nucleic acid, and it provides an antiviral activities to the cells of the same species . It has later been reported that IFN has various effects related to activation of defense mechanism, such as cytostatic effect, antitumor activity, activation of macrophages, and promotion of the killer activity of lymphocytes. IFN is presently classified into four types: α, β, γ and ω, in accordance with their different antigenics. These types of IFN exhibit no significant difference in antiviral activity, but they exhibit differences in their producing cells, inducing conditions, genes, structural characters, receptors, and the like.

Although IFN is a substance having various biological activities, not many studies have been reported regarding its effects on cardiovascular diseases such as vascular lesions or tissue injury by ischaemia, diabetic diseases, collagen disease or wound healing.

An issue of the present invention is to develop, as a curative medicine, a factor that has effects of growth promotion or cell protection of endothelial cells, as a preventive/curative agent for vascular disorders in cardiovascular diseases, diabetic diseases, or collagen diseases, and a traumatic wound healing agent.

To solve this issue, the present invention is directed at providing a industrially and medically useful endothelial cell protective medicine.

### DISCLOSURE OF THE INVENTION

The present invention is an agent for protecting endothelial cells comprising as an effective ingredient IFN .

### BRIEF DESCRIPTION OF THE DRAWINGS

Fig. 1 is a diagram indicating that IFN enhances the production of 2'5' oligoadenylate synthetase by endothelial cells.
Fig. 2 is a diagram indicating that IFN-β increases the survival rate of endothelial cells.
Fig. 3 is a diagram of a study on influence of α, β and γ -type IFNs on the survival rate of endothelial cells.
Fig. 4 shows diagrams indicating the protective effects of IFN-β and VEGF for endothelial cell injury induced by cell injuring substance (t-butyl-hydroperoxide, TBH).
Fig. 5 shows diagrams indicating the protective effects of α and β-type IFNs on endothelial cells injured by peroxide.
Fig. 6 shows diagrams of studies on influence of IFN-β and VEGF on production of various prostaglandins.

### BEST MODES FOR CARRYING OUT THE INVENTION

The diseases concerned in the present invention are those caused by rhexis of endothelial cells, such as angiitis, cardiovascular diseases, diabetic vascular disorders and collagen diseases, or traumatic wound healing , and the like.

As examples of the cardiovascular diseases, ischemic vascular disorders or blood flow insufficiency after heart infarction or brain infarction, arteriosclerotic vascular disorders, arteriosclerotic intima hyperplasia and blood flow insufficiency, peripheral arterial imperforation, vascular restenosis after percutaneous vascular dilatation (PTCA), hypertensive vascular lesion formation, and the like may be cited.

The endothelial cell protective medicine of the present invention may preferably be used for vascular disorders caused by diabetes, that is, great vessel disorders, such as coronary vessel disorders, caused by promotion of arteriosclerotic lesions, and nephropathy and retinopathy. Moreover, it may be used for wound healing of burn, bedsore, racoma, cutting, wound after operation, and chronic skin ulcer, stomatitis chronic skin ulcer in cancer patients subjected to chemotherapy, and angiitis observed in transplants, and the like.

Normally in angiitis, vascular media fibrinoid degeneration and necrosis, and eosinophil leukocytic infiltrate, lymphocytic and monocytic infiltrate, and polymorphonuclear leukocytic infiltrate to vascular walls occur, and vascular intima hyperplasia, clot formation and aneurysm formation are subsequently observed. Since all of these vascular lesions are commonly triggered by endothelial cell injury, IFN is preferably used for such angiitis.

IFN is already approved as a curative medicine for hepatitis, and malignant tumors such as brain tumor, malignant melanoma, and the like. The kinds of IFN used according to the present invention are not particularly limited. Any of α and β-type IFNs, or a consensus-type or a hybrid-type IFNs containing amino acid sequences of the α or β-type, may be used. As for origin, any of natural type, genetically recombinant type or chemically synthesized type may be used. Among these, IFN α and IFN β, particularly, natural-type IFN β, may preferably be used.

For production of natural-type IFN β, fibroblasts or their established cell line are preferably used. If a gene recombination technique is used to prepare IFN, the host cells used may be mammal cells such as the CHO (Chinese hamster ovary) cells, mouse C127 cells and the like, cells of insects such as silk worm, Autographica Californica and the like, microorganisms such as Escherichia coli, Bacillus subtilis, yeast and the like. Furthermore, cells of mouse, rat, hamster, rabbit, goat, sheep, pig, cattle, and the like may be used.

The crude IFN β obtained from the cell culture supernatant, insect extract, bacteria extract, extract from body may be purified and separated by various chromatographies. Any chromatography may be used as long as it provides affinity to IFN. For example, a column containing silicon dioxide (silica) or calcium phosphate as an adsorption material, a column containing heparin, a pigment or a hydrophobic group as a ligand, a metal chelate column, ion exchange column, gel permeability column, and the like may be used.

As stated above, since IFN increases the survival rate of endothelial cells, it can be a preventive agent or a curative agent for various diseases caused by endothelial cell disorders, for example, vascular disorders and blood flow insufficiency after myocardial infarction or brain infarction, arterial sclerosis and vascular restenosis after PTCA, diabetic vascular complications, hypertensive vascular lesions, vasculitis syndrome deriving from collagen diseases, and the like, or a wound healing agent for burn, bedsore, racoma, cutting, wound after operation, and chronic skin ulcer in cancer patients subjected to chemotherapy, and the like.

The IFN used according to the present invention may be orally or non-orally administered directly or in the form of a pharmaceutical composition combining a known carrier or excipient that is pharmacologically allowable.

As concrete examples of the dosage form for oral administration, tablets, pills, capsules, granules, syrups, emulsions, suspensions and the like may be cited. Such a dosage form is produced by a known method and contains a carrier or excipient normally used in the field of formulation. As examples of the carrier and excipient for tablets, lactose, maltose, saccharose, starch, magnesium stearate, and the like may be cited.

As examples of the dosage form for non-oral administration, eye drops, ointments, injections, wet compresses, paints, suppositories, nasal absorptive agents, lung-absorptive agents, skin-absorptive agents, and the like may be cited. A solution formulation can be prepared by a known method, for example, wherein IFN is normally dissolved in a sterilized distilled water prepared for an injection, or suspended in an extract, or, further, emulsified and enclosed in liposomes. A solid formulation can be prepared by a known method, for example, wherein mannitol, trehalose, sorbitol, lactose, glucose, or the like is added as an excipient to IFN, and freeze-dried. A gelatinized formulation can be prepared using freeze-dried products by a known method, for example, wherein IFN is dissolved in a thickener or polysaccharide, such as glycerine, polyethylene glycol, methylcellulose, carboxymethyl cellulose, hyaluronic acid, chondroitin sulfate, and the like.

In any formulation, it is possible to add as a stabilizer, human serum albumin, human immunoglobulin, α-2 macroglobulin, an amide acid, and the like. It is also possible to add as a dispersion agent or an absorption promoting agent, alcohol, sugar alcohol, an ionic surfactant, a non-ionic surfactant, and the like, within a range that does not impede the physiological activity of IFN. A trace metal or an organic acid salt may also be added if necessary.

The IFN according to the present invention may be used as the effective element or one of the effective elements, in IFN singly or combination with another agent with a different acting mechanism. For example, if a substance, for example, vascular endothelial cell growth factor (VEGF), that induces prostacyclin (PGl2) having an endothelial cell protective effect, is used together, a synergistic effect can be expected.

The dosage is suitably determined in accordance with the age and weight of a patient, subject disease of administration, symptom, administration manner, administration route, and the like. The dosage is normally 10-10,000 thousand units/day, and preferably 100-6,000 thousand units/day.

### EXAMPLES

The present invention will be described further in detail with reference to examples. However, the present invention is not limited to these examples.

### EXAMPLE 1

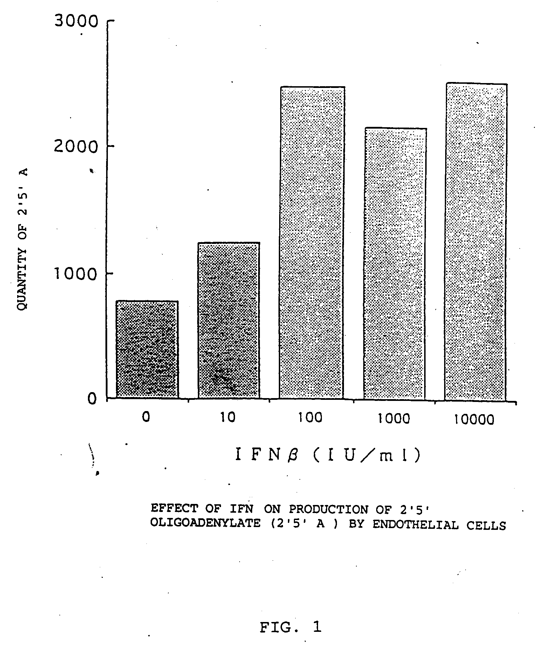

To verify the effect of IFN, the enhancing effect on the synthesis of 2'5' oligoadenylate synthetase by endothelial cells treated with IFN-β was investigated. Endothelial cells separated from a human umbilical cord vein were proliferated in a 25cm² collagen-coated flask using M199 medium containing 10 ng/mL of basic fibroblast growth factor (bFGF) and 10% of fetal calf serum (FCS). After the cells proliferated confluently (number of cells: 3 × 10⁵/flask), the culture solution was replaced with M199 containing 10 to 10,000 units/mL of human natural IFN -β (by Toray Kabushiki Gaisha) and 2% of FCS. Cultivation was further continued at 37 C for 24 hours. After the cells were washed with PBS, the cells were separated from the flask using 0.1% trypsin. After centrifugation, the cells were washed twice with PBS and then subjected to centrifugation at 1000 rpm for 5 minutes, thereby collecting IFN-treated cells. The cells were homogenized with 300 µL of 0.5% CHAPS buffer added thereto, for 30 seconds, and then centrifuged at 3000 rpm for 10 minutes. A supernatant was separated, and the quantity of 2'5' A in the supernatant was measured by Radio-immuno-assay (EIKEN). Results are shown in Fig. 1. The IFN treatment increased the 2'5' A production of the cells, verifying that IFN affected the endothelium.

### EXAMPLE 2

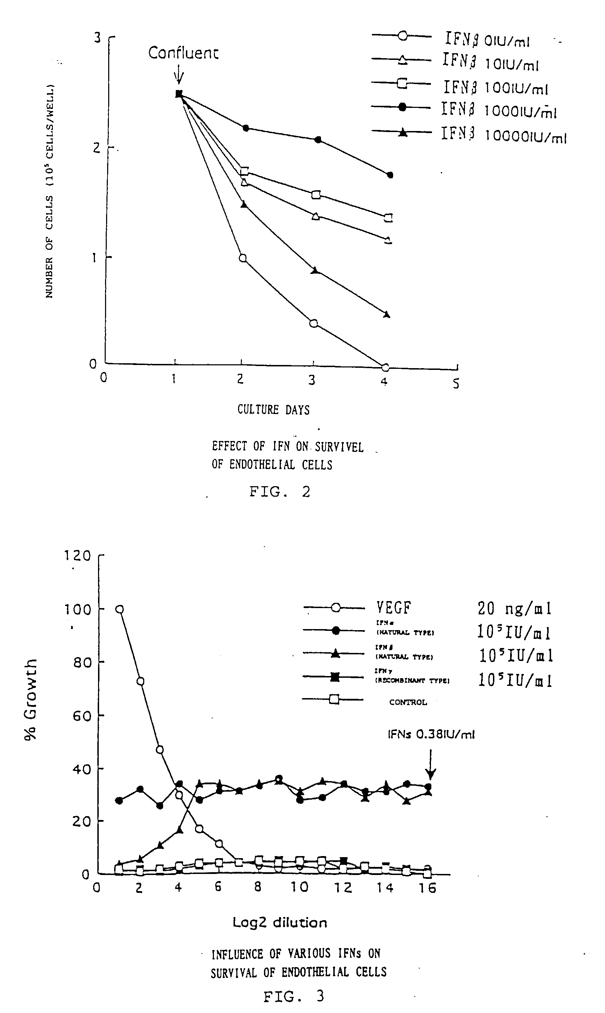

To investigate the effect of IFN on the survival rate of endothelial cells, the following experiment was performed. Endothelial cells separated from human umbilical cord were seeded into a 6-well plate coated with collagen, in an amount of 2 × 10⁵ cells per well. Using M199 medium containing 10 ng/mL of bFGF and 10% of FCS, the cells were cultivated and proliferated for 24 hours. After confluent proliferation of cells was confirmed, the medium was replaced with M199 medium containing 10 to 10,000 units/mL of human natural IFN-β (by Toray Kabushiki Gaisha) and 2% of FCS. Cultivation was further continued for 4 days. The number of cells surviving in low-serum media after confluent proliferation of cells was determined using a hemocytometer. The number of endothelial cells surviving after 4-day cultivation in the absence of IFN was zero. On the other hand, if 10 IU/mL of IFN was added, the survival rate of endothelial cells improved. If 1,000 IU/mL of IFN was added, the cells survived at a rate of 74% relative to the number of cells at the time of addition of IFN. Results are shown in Fig. 2.

### EXAMPLE 3

The influence of α, β, γ-type IFNs on the survival rate of endothelial cells was investigated. A 96-well collagen-coated plate was seeded with endothelial cells in an amount of 5000 cells per well using M199 medium containing 10% of FCS. Two hours after the cells adhered to the plate, the medium was replaced with media prepared by two fold dilution with 2% fcs-added M199 medium from 100 thousand unit/mL of human natural-type IFN-α (by Nippon Chemical Research), human natural-type IFN-β (by Toray) or human recombinant-type IFN-γ (by Genentech). Cultivation was further continued for 48 hours. After the cells were fixed by formalin and stained by crystal violet, the absorbance to 540 nm wavelength was measured to find the influence of these IFNs on the survival rate of the cells. For a control of 100% proliferation, 20 ng/mL of endothelial cell growth factor (VEGF) was used. Results are indicated in Fig. 3. As indicated, although addition of α or β-type IFNs did not cause proliferation of the endothelial cells, the cells was survived at about 30% with 0.38 IU/mL of IFN added.

### EXAMPLE 4

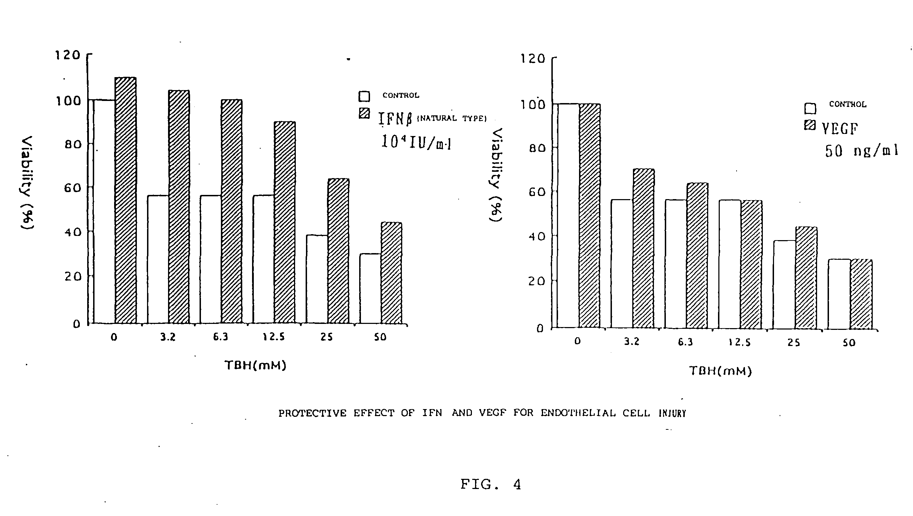

The influence of IFN on endothelial cell injury caused by peroxide was considered as follows. Using TBH (t-butyl-hydroperoxide) as a cell injuring substance, it was first examined whether the survival rate decrease depending on treatment concentration, and then, the influence of IFN under the same conditions was investigated. A 96-well collagen-coated plate was seeded with endothelial cells in an amount of 10 thousand cells per well using M199 medium containing 10% of FCS. Two hours after the cells adhered to the plate, the medium was replaced with a medium containing 2% of FCS and 10 thousand unit/mL of the same IFN as used in Example 1 or 50 ng/mL of growth factor (VEGF) for a control. Cultivation was further continued at 37 C for 12 hours. The cells were then washed with PBS(-). 3.2 to 50 mM TBH dilutions were prepared using PBS containing 0.2 mM CaCl₂. The cells were treated with these dilutions at 37 C for 30 minutes to induce cell injury. The cells treated with TBH were fixed by formalin and, the surviving cells were stained by crystal violet. Then the absorbance to 540 nm was measured, from which the survival rate of the cells relative to the control with no TBH added was determined. Results are indicated in Fig. 4. The endothelial cells exhibited decreases in survival rate depending on the TBH treatment concentration. The survival rate decreases were reduced by IFN. That effect of IFN was stronger than that of VEGF used as control.

### EXAMPLE 5

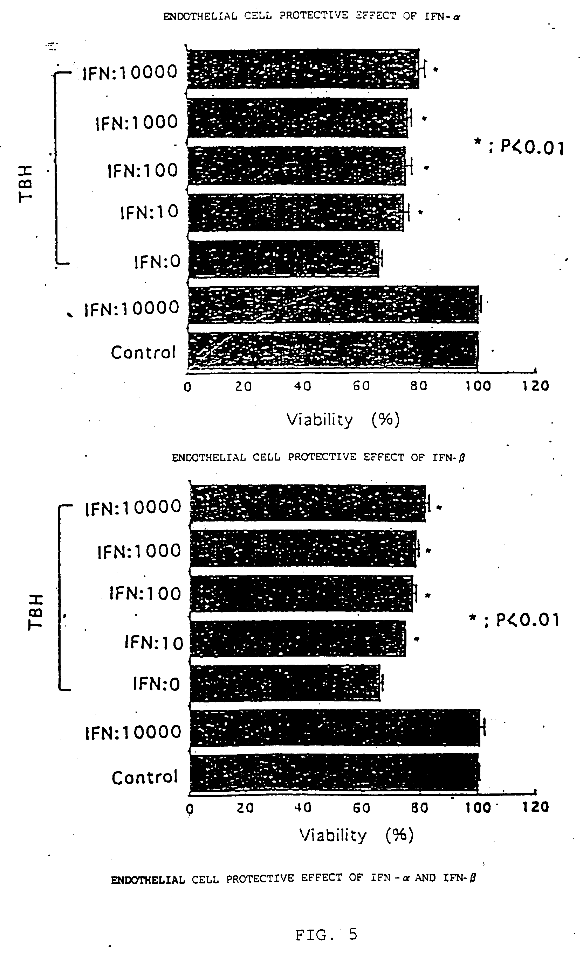

It was investigated whether human natural-type IFN-α (by Nippon Chemical Research) and human natural-type IFN - β (by Toray) had protective effects for endothelial injury induced by cell injuring substance (TBH). Results are indicated in Fig. 5. A 96-well collagen-coated plate was seeded with endothelial cells in an amount of 10 thousand cells per well using M199 medium containing 10% of FCS. Two hours after the cells adhered to the plate, the medium was replaced with a medium containing 2% of FCS and 10-10,000 unit/mL of one of the IFNs. Cultivation was further continued at 37 C for 12 hours. The cells were then washed with PBS(-). 30 mM TBH in PBS containing 0.2 mM CaCl₂ was used to treat the cells at 37 C for 50 minutes. The treated cells were fixed by formalin and, and the surviving cells were stained by crystal violet. Then the absorbance to 540 nm was measured, from which the survival rate of the cells relative to the control without IFN treatment was determined. A representative case of four repeated experiments is indicated in the graphs of Fig. 5. The columns and bars indicate average S.E. (N=4). As for statistic processing, a significant difference from the non-IFN-treated group was tested by Student T Test. It was clear that IFN-α and IFN-β have protective effects for endothelial injury caused by peroxide. In a similar experiment, recombinant-type IFN-γ (by Genentech) showed no significant difference.

### EXPERIMENT 6

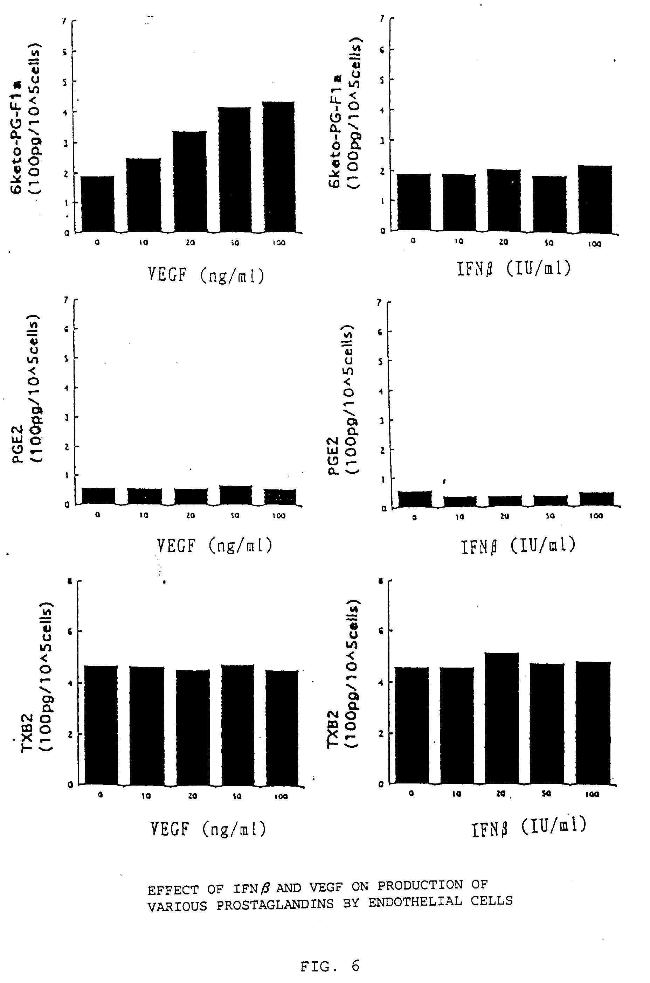

A 24-well plate was seeded with endothelial cells in an amount of 50 thousand cells/hole using M199 medium containing 10% of FCS. After it was observed that the cell adhered and became sufficiently confluent, the medium was replaced with 2%-FCS-added M199 media containing various concentrations of human natural-type IFN-β (by Toray Kabushiki Gaisha) or VEGF for a control. Cultivation was then continued at 37 C for 24 hours. The amounts of various prostaglandins (6keto-PG-F1, that is, a PGl2 metabolite, PGE2 and thromboxane B2) were measured by ELISA. Results are indicated in Fig. 6. The figure shows the mechanism the of endothelial cell protection by IFNs is different from that by VEGF, that is the effect by IFN is not due to the induction of PG12 which is already known to have a protective effect on endothelial cell injury.

### INDUSTRIAL APPLICABILITY

According to the present invention, it becomes possible to provide a preventive agent or a protective agent for various diseases caused by injury of endothelial cells, for example, cardiovascular diseases, diabetic vascular disorders, or vascular disorders in collagen diseases, wound healing, and the like.

## Claims

1. An agent for protecting endothelial cells comprising as an effective ingredient interferon.

2. An agent for protective endothelial cells comprising as an effective ingredient interferon according to claim 1, wherein the agent for protecting endothelial cells is a wound healing agent or a preventive/curative agent for a vascular disorder in a cardiovascular disease, a diabetic vascular disorder or a collagen disease.

3. An agent for protecting endothelial cells comprising as an effective ingredient interferon according to claim 1, wherein the agent for protecting endothelial cells is an angiitis curative agent.

4. An agent for protecting endothelial cells comprising as an effective ingredient interferon according to claim 2, wherein the vascular disorder is an ischemic vascular injury or blood flow insufficiency after myocardial infarction or brain vascular infarction, an arteriosclerotic vascular disorder, arteriosclerotic intima hyperplasia and blood flow insufficiency, peripheral arterial imperforation, vascular restenosis after percutaneous vascular dilatation (PTCA), or hypertensive vascular lesion formation.

5. An agent for protecting endothelial cells comprising as an effective ingredient interferon according to claim 2, wherein the diabetic vascular disorder is a great vessel disorder, nephropathy, retinopathy.

6. An agent for protecting endothelial cells comprising as an effective ingredient interferon according to claim 2, wherein the wound healing agent is a curative agent for burn, bedsore, racoma, cutting, wound after operation, chronic skin ulcer, stomatitis chronic skin ulcer in a cancer patient subjected to chemotherapy, angiitis observed in a transplant.

7. An agent for protecting endothelial cells comprising as an effective ingredient interferon according to claim 1, wherein the interferon is α type or β type.

8. An agent for protecting endothelial cells comprising as an effective ingredient interferon according to claim 1, wherein the interferon is β type.

9. An agent for protecting endothelial cells comprising as an effective ingredient interferon according to claim 1, wherein the interferon is a natural interferon-β type .

10. A composition for protecting endothelial cells, containing an effective amount of interferon in a carrier that is pharmacologically allowable.

11. A method for protecting endothelial cells, comprising administering an effective amount of interferon to a patient who needs protection of endothelial cells.
